# FASCICULE DE BREVET EUROPEEN

(11) **EP 2 260 085 B1**
(45) Date de publication et mention de la délivrance du brevet: **03.10.2012**
(21) Numéro de dépôt: 09731389.4
(22) Date de dépôt: 19.03.2009
(51) Int. Cl.: C09K 11/02, C09K 11/06

(54) **NANOCRISTAUX FLUORESCENTS ENROBES D'UNE COQUILLE INORGANIQUE**
IN EINER ANORGANISCHEN HÜLLE EINGEKAPSELTE FLUORESZIERENDE NANOKRISTALLE
FLUORESCENT NANOCRYSTALS ENCAPSULATED IN AN INORGANIC SHELL

(30) Priorité: 21.03.2008 FR 0851830
(43) Date de publication de la demande: 15.12.2010
(73) Titulaire: Centre National de la Recherche Scientifique - CNRS, 75794 Paris Cedex 16 (FR); Institut National Polytechnique de Grenoble, 38031 Grenoble (FR)
(72) Inventeur: IBANEZ, Alain, F-38500 Voiron (FR); MARCELLIN, Noélie, F-38100 Grenoble (FR); DJURADO, Elisabeth, F-38920 Crolles (FR); PHILIPPOT, Cécile, F-38000 Grenoble (FR)
(74) Mandataire: Novagraaf Technologies
(86) Numéro de dépôt international: PCT/FR2009/000294
(87) Numéro de publication internationale: WO 2009/125086

(56) Documents cités:
- US-A1- 2006 024 436
- OW H ET AL: "Bright and stable core-shell fluorescent silica nanoparticles" NANO LETTERS, ACS, WASHINGTON, DC, US, vol. 5, no. 1, 1 janvier 2005 (2005-01-01), pages 113-117, XP003009815 ISSN: 1530-6984
- DESPORTES ET AL: "Fluorescence lifetime imaging microscopy for in situ observation of the nanocrystallization of rubrene in a microfluidic set-up" CHEMICAL PHYSICS LETTERS, NORTH-HOLLAND, AMSTERDAM, vol. 446, no. 1-3, 14 septembre 2007 (2007-09-14), pages 212-216, XP022247724 ISSN: 0009-2614
- VIRGINIE MONNIER ET AL: "TEM characterization of organic nanocrystals grown in sol-gel thin films" JOURNAL OF NANOPARTICLE RESEARCH ; AN INTERDISCIPLINARY FORUM FOR NANOSCALE SCIENCE AND TECHNOLOGY, KLUWER ACADEMIC PUBLISHERS, DO, vol. 10, no. 1, 4 avril 2007 (2007-04-04), pages 129-139, XP019555338 ISSN: 1572-896X
- PIRJO KORTESUO, MANJA AHOLA, MINNA KANGAS, ILKKA KANGASNIEMI, ANTTI YLI-URPO, JUHA KIESVAAARA: "In vitro evaluation of sol-gel processed spray dried silica gel microspheres as carrier in controlled drug delivery" INTERNATIONAL JOURNAL OF PHARMACEUTICS, vol. 200, 2000, pages 223-229, XP002506820
- ZHAO D ET AL: "Modified spontaneous emission of europium complex nanoclusters embedded in colloidal silica spheres" CHEMICAL PHYSICS LETTERS, NORTH-HOLLAND, AMSTERDAM, vol. 403, no. 1-3, 14 février 2005 (2005-02-14), pages 129-134, XP004727991 ISSN: 0009-2614

## Description

### Domaine technique

La présente invention se rapporte à un procédé de préparation de nanoparticules hybrides organo-minérales, ainsi qu'à des nanoparticules comprenant au moins un nanocristal organique fluorescent enrobé d'une coquille inorganique susceptibles d'être obtenues par ledit procédé et utilisables par exemple comme traceur en imagerie ou comme capteur chimique.

Le procédé de la présente invention comprend les étapes suivantes :
(i) nébuliser un mélange sol-gel susceptible d'être obtenu par un procédé comprenant l'ajout dans au moins un solvant organique, en présence d'eau; d'au moins un composé organique fluorescent et d'au moins un alcoxyde métallique ;
(ii) sécher le mélange sol-gel nébulisé en évaporant le solvant et l'eau présents dans le mélange sol-gel.

Dans la description ci-dessous, les références entre parenthèses **(ref x)** renvoient à la liste des références présentées après les exemples.

### Etat de la technique

Les méthodes optiques, jouent un rôle majeur dans la recherche clinique, en particulier pour le développement de tests de diagnostics et pronostics ou pour le développement de nouvelles thérapies. Notamment, l'angiographie est une technique d'imagerie médicale très utilisée pour le diagnostic médical. L'étude des vaisseaux sanguins permet en effet d'identifier des pathologies vasculaires, des anomalies liées à l'écoulement du sang, de repérer le trajet des vaisseaux et ainsi de mettre au point un traitement adapté ou de préparer au mieux une intervention chirurgicale. En outre, c'est une technique largement utilisée en neurosciences.

C'est généralement la fluorescence d'un colorant injecté qui, à la suite d'une excitation lumineuse, sert à imager les vaisseaux sanguins. Le protocole classique consiste en l'injection simultanée de deux colorants, un de faible masse moléculaire est choisi pour son aptitude à diffuser à travers la barrière hémato encéphalique (parois des vaisseaux sanguins) alors que l'autre, avec un poids moléculaire nettement plus élevé, est au contraire destiné à la seule coloration intravasculaire (non diffusible au travers des parois de vaisseaux).

Les propriétés souhaitables du colorant diffusible sont largement satisfaites par un large éventail de colorants commerciaux, ce qui n'est en revanche pas le cas pour la coloration intravasculaire. En effet, les colorants destinés à une coloration intravasculaire nécessitent des poids moléculaires élevés, or, la seule solution commerciale existante pour la coloration intravasculaire consiste à greffer sur un agrégat polymérique volumineux, tel que le dextran ou l'albumine, une molécule fluorescente. Cependant, il est difficile de greffer un nombre important de fluorophores sur un même agrégat polymérique. Ceci entraine une forte dilution des chromophores d'où un contraste en imagerie insuffisant lors d'études de vaisseaux en profondeur. Ainsi, les techniques actuelles permettent d'imager les vaisseaux de faible profondeur (de l'ordre du mm) mais restent insuffisantes pour un examen profond des capillaires lésés et perméables qui sont généralement rencontrés au sein des tumeurs.

En effet, l'absorption et la diffusion de la lumière d'excitation sont responsables de cette limitation en profondeur. Une excitation par microscopie à balayage laser dans la zone spectrale 650-1000 nm rend négligeable la perte par absorption à travers l'os, la dure-mère et la matière blanche et grise du cerveau.

Pour pallier le problème de la diffusion, une solution serait d'augmenter la puissance du laser. Cependant, cette voie, généralement adoptée pour l'imagerie ex vivo, n'est pas tolérable in vivo car elle engendre un échauffement local qui perturbe la mesure et qui peut également provoquer des lésions irréversibles. Par exemple, une valeur limite de puissance laser tolérable in vivo, déterminée empiriquement, est de quelques mW au point focal de l'objectif pour une longueur d'onde d'excitation de 800 nm.

Une autre solution à la limitation en profondeur d'imagerie consiste à utiliser des traceurs plus lumineux, soit en augmentant la concentration locale en fluorophores, soit en utilisant des fluorophores avec des rendements de luminescence élevés.

Ainsi, des nanocristaux semi-conducteurs ont été développés, tels que décrits par exemple dans l'article D.R. Larson et al. Sciences 300 (2003), 1434-1436 **(ref 1)**.

Cependant, les nanocristaux semi-conducteurs sont difficiles à synthétiser en grande quantité car leur synthèse est longue, fastidieuse et très coûteuse. En effet, la synthèse est réalisée en plusieurs étapes : dans un premier temps, il s'agit d'obtenir un coeur fluorescent de petite taille, c'est à dire de 2 à 5 nm au maximum. Dans une deuxième étape, il est impératif de former, par croissance hétéro-épitaxiale, une coquille d'un semi-conducteur à plus grand gap et ceci pour pallier les problèmes de clignotement. En effet, les problèmes de clignotement sont dus à des défauts de surface qui engendrent des états non fluorescents transitoires et aléatoires. Par exemple, J.S. Steckel et al. Ang. Chem. Inter. Ed. 43 (2004), 2154-2158 **(ref 2),** décrit des nanocristaux obtenus par croissance d'une coquille de ZnS. De plus, ces nanocristaux semi-conducteurs ne sont pas biocompatibles et ne sont pas non plus dispersables en solution physiologique. Ainsi, la synthèse nécessite une troisième étape qui consiste à fonctionnaliser ces nanocristaux semi-conducteurs, par exemple, par une encapsulation en micelles phospholipides comme décrit dans l'article B. Dubertret et al. Sciences 298 (2002), 1759-1762 **(ref 3)**.

Un autre inconvénient concerne la perte d'efficacité des nanocristaux semi-conducteurs lorsqu'ils sont fortement excités du fait de l'interaction entre porteurs, telle que l'effet Auger.

Par ailleurs, d'autres travaux de recherche fondamentale en cours portent sur l'élaboration par chimie sol-gel en solution de particules de silice dans lesquelles sont dispersées ou greffés à la matrice silicatée des molécules organiques fluorescentes, comme décrit dans le document de Larson et al. Nano Letters, vol 5 (2005), 1, 113-117 **(ref 4).** Cependant, pour ce type de traceur biologique, la concentration en molécules fluorescentes reste limitée. De plus, on ne bénéficie pas ici de l'état cristallin de la partie organique fluorescente qui stabilise d'autant plus chimiquement et photochimiquement les fluorophores organiques. En effet, il ne s'agit pas ici de nanocristaux de fluorophores organique au niveau du coeur de la particule.

De nombreuses applications bio-médicales, comme les techniques d'imagerie, nécessitent des quantités importantes de fluorophore afin d'obtenir une coloration du circuit sanguin complet. Celles-ci présentent notamment un intérêt considérable pour le développement de nouvelles thérapies pour l'homme. Ces techniques sont généralement testées dans un premier temps sur les souris et les rats et nécessitent environ 10mg de fluorophore par souris et beaucoup plus pour les rats, ce qui est un sérieux handicap pour les nanocristaux semi-conducteurs pour de simples raisons de prix.

Les applications bio-médicales nécessitent également des nanocristaux avec des distributions de taille très étroites et de très bonnes stabilités afin de visualiser plusieurs fois une même zone in vivo et de pouvoir observer des évolutions au cours du temps (mécanismes de migration des traceurs, évolutions thérapeutiques, etc.).

Il existe donc un réel besoin de disposer de nanocomposés luminescents améliorés permettant de résoudre en tout ou partie les problèmes évoqués ci-dessus, notamment en termes de stabilité et de résistance, par exemple thermique, mécanique, chimique, biochimique et/ou photochimique.

Il existe également un réel besoin pour des nanocomposés luminescents présentant de bonnes propriétés optiques, comme la fluorescence, la photostabilité, la gamme de transparence, mais également de bonnes propriétés de surface, comme la rugosité qui a une influence sur les propriétés optiques ou le caractère hydrophile nécessaire pour la biocompatibilité ou la faculté des composés à être dispersables en solutions physiologiques.

En outre, il existe un réel besoin de disposer d'un procédé rapide et simple de préparation de ces nanocomposés optimisés applicable à la synthèse en grandes quantités et permettant en outre de réduire les coûts de fabrication, d'améliorer les rendements et les quantités produites.

### Description de l'invention

La présente invention a précisément pour but de répondre à ces besoins et inconvénients de l'art antérieur en fournissant un procédé de préparation de nanoparticules comprenant au moins un nanocristal organique fluorescent enrobé d'une coquille inorganique, ledit procédé comprenant les étapes suivantes :
(i) nébuliser un mélange sol-gel susceptible d'être obtenu par un procédé comprenant l'ajout dans au moins un solvant organique, en présence d'eau, d'au moins un composé organique fluorescent et d'au moins un alcoxyde métallique de formule (I) suivante :

   R¹ₓM(OR²)_{y} Formule (I)

   dans laquelle :
   - M est un métal choisi dans le groupe comprenant Si, Ti, Zr, Sn, B, Al et Y, par exemple Si.
   - x est un nombre entier allant de 0 à 2, par exemple 0 ou 1 ;
   - y est un nombre entier allant de 1 à 6, par exemple 3 ou 4 ;
   - x+y correspond au nombre de coordination du métal M ;
   - R¹ et R² représentent indépendamment un radical organique compatible avec la chimie sol-gel ;
(ii) sécher le mélange sol-gel nébulisé en évaporant le solvant et l'eau présents dans le mélange sol-gel.

Selon un mode de réalisation particulier, l'invention se rapporte à un procédé de préparation de nanoparticules comprenant un coeur cristallin organique fluorescent enrobé d'une coquille inorganique, ledit procédé comprenant les étapes suivantes :
(i) nébuliser un mélange sol-gel susceptible d'être obtenu par un procédé comprenant l'ajout dans au moins un solvant organique, en présence d'eau, d'un composé organique fluorescent et d'au moins un alcoxyde métallique de formule **(I)** suivante :

   R¹ₓM(OR²)_{y} Formule (I)

   dans laquelle :
   - M est un métal choisi dans le groupe comprenant Si, Ti, Zr, Sn, B, Al et Y, par exemple Si.
   - x est un nombre entier allant de 0 à 2, par exemple 0 ou 1 ;
   - y est un nombre entier allant de 1 à 6, par exemple 3 ou 4 ;
   - x+y correspond au nombre de coordination du métal M ;
   - R¹ et R² représentent indépendamment un radical organique compatible avec la chimie sol-gel ;
(ii) sécher le mélange sol-gel nébulisé en évaporant le solvant et l'eau présents dans le mélange sol-gel.

La chimie sol-gel et ses divers modes de mise en oeuvre sont connus de l'homme de métier, ont fait l'objet de nombreux rapports et publications, et ne seront pas développés dans le cadre du présent brevet. On citera par exemple, le livre de référence dans le domaine de C.J. Brinker et G.W. Scherer, Sol-Gel Science, The Physics and Chemistry of SolGel Processing, Academic Press, New York, 1990 (ref 5), l'article de Avnir et al., J. Non. Cryst. Solids, 1985, 74, 395-406 (ref 6) ou encore Sanchez et al., New J. Chem., 1994, 18, 1007-1047 (ref 7).

L'homme du métier pourra sélectionner R¹ et R² à la lumière des ouvrages précités et de ses connaissances générales dans le domaine de la chimie sol-gel. Par exemple, R¹ et R² peuvent représenter indépendamment un radical alkyle en C₁ à C₁₅, hétéroalkyle C₁ à C₁₅, aryle en C₆ à C₂₅ ou hétéroaryle en C₄ à C₂₅, les radicaux R¹ et R² étant indépendamment éventuellement substitués par un ou plusieurs groupes R indépendamment choisis dans le groupe comprenant un radical alkyle en C₁ à C₁₀, hétéroalkyle C₁ à C₁₀; aryle en C₆ à C₁₀ ou hétéroaryle en C₄ à C₁₀ ; F ; Cl ; Br; I; -NO₂; -CN ; ou une fonction -GR^{G1} dans laquelle G est -O-, -S-, -NR^{G2}-, -C(=O)-, -C(=O)O-, -C(=O)NR^{G2}-, où chaque occurrence de R^{G1}, R^{G2} et R^{G3} est indépendamment des autres occurrences de R^{G1} un atome d'hydrogène, ou un radical alkyle en C₁ à C₁₀, hétéroalkyle C₁ à C₁₀; aryle en C₆ à C₁₀ ou hétéroaryle en C₄ à C₁₀ ; ou bien, lorsque G représente -NR^{G2}-, R^{G1} et R^{G2} conjointement avec l'atome d'azote auquel ils sont liés forment un hétérocycle ou un hétéroaryle éventuellement substitué ;

On entend par radical « alkyle » au sens de la présente invention, un radical carboné linéaire ou ramifiée, cyclique ou acyclique, saturé ou insaturé. Il peut par exemple s'agir de C₁₋₁₅alcane; C₂₋₁₅alcène; C₂₋₁₅alcyne; C₃₋₁₅cycloalkyle. Selon l'invention, le radical alkyle peut comprendre 1 à 15 atomes de carbone, par exemple 1 à 10 atomes de carbone, par exemple 1 à 6 atomes de carbone. On entend par « hétéroalkyle » au sens de la présente invention, un radical alkyle, tel que défini précédemment, dans lequel au moins un atomes de carbone est remplacé par un hétéroatome, notamment choisi dans le groupe comprenant l'oxygène, le soufre, l'azote, le phosphore et le bore. Il peut par exemple s'agir d'un hétéroalcane, d'un hétéroalcène, d'un hétéroalcyne, d'un hétérocycle, d'un alkoxy, d'un alkylthio, d'une alkylamine, d'un alkylamide, d'une alkylimine, d'un alkylimide, d'un alkylester, d'un alkyléther, etc. Selon l'invention, le radical hétéroalkyle peut comprendre 1 à 15 atomes de carbone, par exemple 1 à 10 atomes de carbone, par exemple 1 à 6 atomes de carbone.

On entend par « aryle » au sens de la présente invention, un radical comprenant au moins un cycle satisfaisant la règle d'aromaticité de Hückel. Ledit aryle peut être mono- ou polycyclique, fusionné ou non. Il peut s'agir par exemple d'un phényle, d'un benzyle, d'un tolyle, etc. Selon l'invention, le radical aryle peut comprendre 6 à 25 atomes de carbone, par exemple 6 à 18 atomes de carbone, par exemple 6 à 10 atomes de carbone. On entend par « hétéroaryle » au sens de la présente invention, un radical aryle, tel que défini précédemment, dans lequel au moins un atomes de carbone est remplacé par un hétéroatome, notamment choisi dans le groupe comprenant l'oxygène, le soufre et l'azote.

On entend par mélange « sol-gel » au sens de la présente invention un mélange comprenant initialement au moins un alcoxyde métallique de formule (I) en présence d'eau, au moins un solvant organique et au moins un composé organique fluorescent, ledit mélange étant initialement sous la forme d'une solution et produisant un gel par réaction d'hydrolyse et de polycondensation. En effet, la présence d'eau permet d'initier des réactions d'hydrolyse et de condensation des alcoxydes métalliques formant un réseau inorganique conduisant à des gels puis des xérogels. Ces xérogels conduisent à un réseau solide inorganique structuré d'oxydes métalliques. La chimie sol-gel est par exemple citée dans le brevet FR n° 2 853 307 **(ref 8)**.

Dès le mélange sol-gel constitué, les réactions suivantes d'hydrolyse (1) et de condensation (2) des alcoxydes métalliques peuvent par exemple se produire, conduisant à des « polymères inorganique » d'oxydes métalliques :

(1) R¹ₓM(OR²)_{y} + H₂O → R¹ₓ(OR²)_{y-1}M(OH) + HOR²

(2) R¹ₓ(OR²)_{y-1}M(OH) + R¹ₓ(OR²)_{y-1}M(OH) → R¹ₓ(OR²)_{y-1}M-O-M(OR²)_{y-1}R¹ₓ + H₂O

où R¹, R², x et y sont tels que définis précédemment.

Avantageusement, le mélange sol-gel du procédé de l'invention peut être homogène et stable chimiquement et mécaniquement. Par exemple il peut être stable pendant plusieurs mois voire plusieurs années ce qui présente un fort avantage pour le développement industriel du procédé de l'invention.

On entend par « alcoxydes métalliques », également appelés « précurseurs sol-gel » au sens de la présente invention, tout composé métallique de formule (I). Selon l'invention, on peut utiliser tout alcoxyde métalliques connu de l'homme du métier dans la mesure où l'on peut obtenir un réseau sol-gel polymérique interconnecté. Le lecteur pourra se référer par exemple au livre de référence C.J. Brinker et G.W. Scherer, Sol-Gel Science, The Physics and Chemistry of SolGel Processing, Academic Press, New York, 1990. **(ref 5).**

On entend par « composé organique fluorescent », également appelé « fluorophore » au sens de la présente invention, tout composé organique connu de l'homme du métier qui soit fluorescent dans l'état cristallin. Eventuellement, le composé organique fluorescent peut être initialement introduit sous forme de poudre microcristalline, et dissous totalement dans le solvant afin d'obtenir une solution parfaitement homogène au niveau moléculaire.

Selon l'invention, le composé organique fluorescent peut être par exemple choisi dans le groupe comprenant la famille des polyaromatiques, par exemple le rubrène ou le tétracène, la famille des stilbènes, par exemple le cyano-méthoxy-nitro-stilbène (CMONS) ou le diéthyl-amino-nitro-stilbène, la famille des naphtilimides, la famille des rhodamines, par exemple la rhodamine B, la famille des diarylméthanes, par exemple l'auramine O, la famille des pérylènes diimides, les dérivés du dipyrrométhène difluorure de bore, les complexes de terre rares, par exemple les complexes de Ruthénium, d'Osmium, d'Iridium, d'Europium, d'Ytterbium, d'Erbium, de Néodyme. Dans un mode de réalisation particulier, le composé organique peut être le rubrène ou le CMONS.

On entend par composé ou nanocristal « fluorescent » au sens de la présente invention, un composé ou un nanocristal ayant la propriété d'émettre, suite à une excitation lumineuse, un rayonnement électromagnétique dans le domaine de la lumière visible ou dans le proche infrarouge (IR). Par exemple, le composé ou le nanocristal fluorescent peut émettre un rayonnement électromagnétique aux longueurs d'onde de 400 à 1200 nm, qui correspond à la fenêtre de relative transparence des tissus vivants. Par exemple, le composé ou le nanocristal fluorescent peut émettre un rayonnement électromagnétique aux longueurs d'onde de 400 à 1000 nm, par exemple de 550 à 800 nm, qui correspond à une fluorescence dans le rouge et le proche infrarouge. En effet, dans ces domaines de longueur d'onde, les tissus biologiques sont le plus transparents ce qui constitue un avantage pour des applications en imagerie.

On entend par « solvant » au sens de la présente invention tout solvant connu de l'homme du métier compatible avec le procédé sol-gel de la présente invention, c'est à dire pouvant rendre miscibles les alcoxydes métalliques et l'eau d'hydrolyse afin d'obtenir des solutions homogènes et peu visqueuses pour qu'elles puissent être nébulisées. Avantageusement, le solvant permet dissoudre totalement le fluorophore.

Selon l'invention, le solvant présent dans le mélange sol-gel peut être introduit initialement en quantité élevée par rapport à la quantité initiale d'alcoxyde métallique. Par exemple, le mélange sol-gel peut comprendre initialement un nombre de mole de solvant 20 à 200 fois supérieur au nombre de mole initial d'alcoxyde métallique, de préférence 50 à 100 fois supérieur.

Selon l'invention, l'eau présente dans le mélange sol-gel peut être introduite initialement en faible quantité, celle-ci étant suffisante pour initier les réactions conduisant à la polycondensation du réseau inorganique, ou en quantité supérieure à la quantité initiale d'alcoxyde métallique. Par exemple le mélange sol-gel peut comprendre initialement un pourcentage molaire d'eau par rapport au nombre de fonctions alcoxydes (-OR²) de 10 à 400%, de préférence de 20 à 100%, de manière plus préférée de 50 à 100 %.

Selon l'invention, le mélange sol-gel peut comprendre initialement un nombre de mole de fluorophore 100 à 5 fois inférieur au nombre de mole initial d'alcoxyde métallique, de préférence 20 à 10 fois inférieur.

Selon un mode de réalisation particulier de l'invention, le procédé de l'invention peut permettre de préparer des nanoparticules comprenant au moins un nanocristal organique fluorescent enrobé d'une coquille inorganique, ledit au moins un nanocristal n'émergeant pas à la surface de la coquille.

Selon un autre mode de réalisation de l'invention, ladite nanoparticule peut comprendre un seul nanocristal organique fluorescent enrobé d'une coquille inorganique.

Le procédé de la présente invention peut comprendre en outre, avant l'étape de nébulisation (i), une étape préalable (0) de préparation du mélange sol-gel à nébuliser. L'étape (0) de préparation du mélange sol-gel peut comprendre :
- une étape (0a) de préparation d'un mélange initial, par exemple en mélangeant dans un solvant au moins un composé organique fluorescent et au moins un alcoxyde métallique en présence d'eau ; et éventuellement,
- une étape de stockage (0b) du mélange initial pendant une durée d afin de laisser réagir le mélange initial.

Selon l'invention, la durée d peut être inférieure à plusieurs mois voire plusieurs années. Par exemple, la durée d peut être de 1 jour à 1 année, par exemple de 7 jours à 21 jours. En effet, le stockage du mélange permet de laisser vieillir le mélange afin de bien avancer les réactions d'hydrolyse et de condensation des alcoxydes métalliques.

Selon l'invention, l'étape (0) de préparation du mélange sol-gel à nébuliser peut comprendre l'ajout d'un acide au mélange initial. En effet, l'acide, en abaissant le pH du mélange, permet de favoriser l'obtention de longues chaînes inorganiques qui sont favorables à la formation d'une coquille inorganique dense autour des cristaux organiques. Selon l'invention, le pH du mélange peut être de 1 à 7, de préférence de 1 à 2.

On entend par « acide » au sens de la présente invention les acides de Bronsted, minéraux ou organiques. Parmi les acides utilisables, on peut citer par exemple l'acide chlorhydrique, l'acide nitrique ou l'acide acétique.

Selon un mode particulier de réalisation de l'invention, le procédé de la présente invention peut être réalisé avec une ou plusieurs des conditions suivantes :
(a) le composé organique fluorescent peut être par exemple choisi dans le groupe comprenant la famille des polyaromatiques, la familles des stilbènes, la familles des naphtilimides, la famille des rhodamines, la famille des diarylméthanes, la famille des pérylènes diimides, les dérivés du dipyrrométhène difluorure de bore, les complexes de terre rares ;
(b) l'alcoxyde métallique peut être par exemple choisi dans le groupe comprenant le tétraméthoxysilane (TMOS, Si(OCH₃)₄), le tétraéthoxysilane (TEOS, Si(OC₂H₅)₄), le méthyltriméthoxysilane (MTMOS, CH₃Si(OCH₃)₃), l'éthyltriéthoxysilane (ETEOS, C₂H₅Si(OC₂H₅)₃), le 1,2-bis(triméthoxysilyl)ethane (TMSE), le 3-glycidoxypropyl)trimethoxysilane (GPTMS), ou un mélange de ceux-ci ;
(c) le solvant peut être par exemple choisi dans le groupe comprenant les alcools R^{S}-OH où R^{S} est un radical alkyle en C₁ à C₆, les cétones R^{S1}-C(=O)-R^{S2}, les éthers R^{S1}-O-R^{S2}, où R^{S1} et R^{S2} sont indépendamment des radicaux alkyles en C₁ à C₆, le tétrahydrofurane, l'acétonitrile, le diméthylformamide, le toluène, le diméthylsulfoxyde, le dioxane, l'acétone, l'acide acétique, l'acide formique, le dichlorométhane, le chloroforme, le dichloroéthane, l'acétate d'éthyle, le diéthyléther ou un mélange de ceux-ci ;

Selon un mode plus particulier de réalisation de l'invention, le composé organique fluorescent peut être choisi dans le groupe comprenant le rubrène, le tétracène, le cyano-méthoxy-nitro-stilbène (CMONS), le diéthylamino-nitro-stilbène, la rhodamine B, l'Auramine O, et les complexes d'Europium, d'Ytterbium, d'Erbium, de Néodyme.

Selon l'invention, l'étape (i) de nébulisation du mélange sol-gel peut être réalisée sous vide ou sous atmosphère de gaz de façon à générer, à partir du mélange sol-gel, un aérosol. L'étape (i) de nébulisation peut être effectuée par exemple par ultrasons, par nébulisation pneumatique, par électrospray, par injection, par buses de type imprimante jet d'encre, etc. De préférence, l'étape (i) de nébulisation du mélange sol-gel peut être effectuée par ultrasons, par exemple par excitation piézoélectrique de la solution. En effet, cette technique présente l'avantage de générer des aérosols constitués de gouttelettes présentant des distributions de taille étroites.

Le procédé de la présente invention peut comprendre en outre, après l'étape (i) et avant l'étape (ii), une étape de transport (iii) de l'aérosol sous flux de gaz vers une zone de séchage d'un réacteur, l'étape (ii) étant réalisée dans ladite zone de séchage. Avantageusement, le gaz utilisé est un gaz sec et dépoussiéré. Dans tous les modes de réalisation décrits dans la présente invention, parmi les gaz secs utilisables (par exemple pour le transport et/ou le séchage de l'aérosol), on peut citer par exemple l'air, l'azote, l'argon, l'hélium, le CO₂. Avantageusement, l'étape (iii) de transport de l'aérosol peut être réalisée sous flux laminaire afin de réduire la coalescence des gouttelettes.

Selon l'invention, l'étape (ii) de séchage du mélange sol-gel consiste à évaporer le solvant et l'eau présents dans le mélange sol-gel. L'eau présente dans le mélange peut être l'eau introduite au cours de l'étape (0) et/ou produite lors des réactions de polycondensation des alcoxydes métalliques. L'acide éventuellement ajouté au mélange au cours de l'étape (0) peut également être évaporé au cours l'étape (ii). Selon l'invention, l'étape (ii) de séchage du mélange sol-gel nébulisé permet, en évaporant le solvant et l'eau contenus dans les gouttelettes, de former le réseau inorganique par polycondensation, conduisant tout d'abord à la formation de la coquille inorganique en périphérie et en fin d'évaporation de solvant à la cristallisation organique au coeur de la nanoparticule. En effet, en début d'évaporation du solvant, la formation d'une « peau inorganique » est plus dense en surface de la gouttelette qu'en profondeur puisque l'évaporation du solvant est plus élevée à la périphérie de goutte. Puis, lorsque l'évaporation du solvant se poursuit, la sursaturation de la phase organique augmente très rapidement dans le solvant résiduel tandis que la probabilité de nucléation devient nettement plus élevée au coeur des gouttes qu'à leur surface. En effet, cette probabilité de nucléation *dP_{N}* est directement proportionnelle au volume des pores du réseau inorganique en cours de polycondensation : *dP_{N} = J.V.dt*, où J est le taux de nucléation (en nucléi par seconde), V est le volume dans lequel il y a nucléation (en cm³) et dt l'intervalle de temps (en secondes). La nucléation des nanocristaux organiques se produit donc au centre des gouttes là où le volume des pores et la probabilité de nucléation sont plus élevés. Durant la fin d'évaporation du solvant, la croissance des nanocristaux organiques repousse vers la périphérie le réseau sol-gel qui n'est pas encore très rigide. Ainsi, on obtient après élimination totale du solvant des particules hybrides constituées d'un coeur nanocristallin organique enrobé d'une coquille inorganique amorphe et dense. Cette hypothèse initiale, de la croissance confinée de nanocristaux au coeur de la particule inorganique en cours de densification, a été confirmé par différentes techniques de caractérisation, certaines d'entre elles étant présentées dans les exemples ci-dessous.

Avantageusement, l'étape (ii) de séchage peut être effectuée rapidement, par exemple en un temps inférieur à 1 minute, par exemple de 1 à 60 secondes, de préférence de 10 à 20 secondes. En effet, un séchage rapide permet d'éviter la coalescence des gouttelettes. Cependant, une évaporation trop instantanée du solvant (inférieure à la seconde) conduit à une mauvaise cristallinité du coeur organique.

Selon l'invention, l'étape (ii) de séchage peut être réalisée avec au moins une des conditions suivantes :
- à des conditions de température et de pression permettant l'évaporation du solvant et de l'eau présents dans le mélange sol-gel;
- sous un flux de gaz sec;
- en présence d'un matériau permettant de capter le au moins un solvant.

Les conditions de température et de pression peuvent également permettre éventuellement l'évaporation de l'acide susceptible d'être présent dans le mélange sol-gel.

Les conditions de température et de pression utilisables dépendent de la température d'ébullition du solvant ou du mélange de solvant utilisé. En outre, les conditions de température doivent être inférieures à la température de dégradation des nanocristaux organiques. Par exemple, l'étape (ii) de séchage peut être réalisée à une température inférieure à 300°C, de préférence de 50 à 150 °C. En outre, l'étape (ii) de séchage peut être réalisée sous vide, sous pression réduite ou sous atmosphère de gaz sec. Par exemple, l'étape (ii) de séchage peut être réalisée par élévation de température couplée à une dilution dans un gaz sec.

On entend par « matériau capable de capter le au moins un solvant » un matériau absorbant, un adsorbant ou un desséchant. Ledit matériau peut être par exemple disposé autour d'un tube poreux dans lequel circule l'aérosol en cours de séchage. Parmi les matériaux utilisables, on peut citer par exemple des charbons actifs pour piéger des solvants organiques.

Le procédé de la présente invention peut comprendre en outre, après l'étape (ii), une étape de collecte (iv) des nanoparticules en sortie de la zone de séchage. Par exemple, l'étape (iv) de collecte peut être réalisée au moyen d'une plaque de verre, d'un tamis, d'un filtre, d'une membrane poreuse, d'une cartouche filtrante associée à une pompe. Le mode de collection des particules peut être optimisé par exemple en utilisant en bout de réacteur un filtre électrostatique qui consiste à collecter des particules chargées sous haute tension. En effet, un tel filtre permet de faire passer les particules entre deux électrodes soumises à une forte différence de potentiel (de plusieurs kV). Les particules se chargeant sur l'une des électrodes peuvent ainsi être récoltées. Ce mode de collecte par filtre électrostatique est par exemple décrit pour la collecte de nanoparticules d'oxyde d'yttrium dans le document de Joffin et al., J. of Luminescence 113 (2005), 249-257 **(ref 9).** Un autre mode de collection peut être une cartouche filtrante associée à une pompe (par exemple une pompe à membrane) afin de créer une dépression en sortie du réacteur, juste après la zone de séchage et de récupérer à l'aide d'une cartouche filtrante les particules après séchage. En optimisant ces deux systèmes de collecte de particules, le rendement du procédé peut atteindre des taux proches de 100%. Cette deuxième voie devrait favoriser un écoulement laminaire de l'aérosol et réduire ainsi la coalescence de gouttes qui entraînent un élargissement de la distribution de taille des particules. Selon un mode de réalisation particulier de l'invention, l'étape (iv) de collecte peut permettre en outre de sélectionner des tailles particulières de nanoparticules.

Le procédé de la présente invention conduit ainsi à des nanoparticules présentant une phase organique au centre et une phase inorganique en périphérie. Autrement dit, il s'agit de nanoparticules de géométrie coeur-coquille, dans laquelle le coeur est constitué d'un composé organique flurorescent cristallin (« coeur cristallin »), et la coquille est une couche inorganique. Par « phase organique » on entend au sens de la présente invention la partie de la nanoparticule constituant le nanocristal fluorescent confiné au centre. Par « phase inorganique », on entend au sens de la présente invention la partie de la nanoparticule constituant la coquille inorganique.

Ainsi, le procédé de préparation de l'invention permet l'obtention de nanoparticules hybrides pures, très lumineuses, de distribution de taille homogène. Ce procédé est une méthode très simple et douce car effectuée à une température proche de l'ambiante. En effet, l'élaboration des nanoparticules est réalisée en une seule étape réactionnelle à partir de solutions stables et homogènes qui sont faciles à préparer. De plus, cette méthode est générique car elle est applicable à tout fluorophore organique soluble et recristallisable dans le solvant organique compatible avec le procédé sol-gel.

En outre, le procédé de préparation de l'invention a l'avantage de permettre l'obtention de ces nanoparticules en grande quantité et en continu, en un nombre réduit d'étapes, avec des rendements élevés et un coût de production faible. Par exemple, le procédé de préparation de l'invention permet l'obtention de ces nanoparticules avec un rendement d'au moins 25%, par exemple d'au moins 50%, par exemple supérieur à 70%, par exemple supérieur à 80%, par exemple supérieur à 90%, par exemple proche de 100%. Ainsi, le procédé de l'invention est directement transposable au niveau industriel.

De plus, le procédé de la présente invention permet aisément de moduler la taille des nanoparticules ce qui a un impact sur leur furtivité en milieu biologique (notamment des tailles inférieures à 80-100nm sont favorables pour l'utilisation de ces nanoparticules comme traceur en imagerie) et rend possible l'étude de migration de médicaments « in vivo ». En effet, la taille des particules peut être gouvernée par exemple par la taille des gouttelettes formées dans l'aérosol et surtout plus aisément par la proportion de solvant organique par rapport aux quantités de fluorophores et d'alcoxyde du mélange initial. Par exemple, plus les précurseurs sol-gel et le fluorophore sont dilués dans le solvant, plus la taille des particules obtenues après séchage est petite.

Le procédé de l'invention permet également de contrôler l'épaisseur de la coquille ou la taille du nanocristal afin d'optimiser les propriétés optiques en fonction du type d'application envisagé, par exemple l'angiographie cérébrale.

Notamment, les inventeurs ont mis en évidence les principaux paramètres expérimentaux (rapports molaires d'alcoxydes métallique, solvant, chromophore organique et eau, formation d'aérosol et conditions de séchage...) pour le contrôle des conditions de nanocristallisation, c'est à dire la nucléation et la croissance confinée des molécules organiques au sein de la matrice sol-gel. La maîtrise des conditions de nanocristallisation conduisent à un bon rapport diamètre des nanocristaux / diamètre de la particule hybride, en jouant sur la proportion relative alcoxyde / phase organique. Selon un mode de réalisation particulier de l'invention, le rapport diamètre des nanocristaux / diamètre de la nanoparticule peut être supérieur à 0,5.

En outre, cette méthode d'élaboration requiert un parfait contrôle de l'aérosol avec des distributions étroites de taille de gouttelettes, un transport sous flux laminaires de l'aérosol afin de réduire au maximum la coalescence des gouttes, etc. Pour cela, différents types de réacteurs de nébulisation de solution peuvent être utilisés selon la méthode de nébulisation souhaitée (par exemple ultra-sons, pneumatique, électrospray, buses de type imprimante jet d'encre comme cité précédemment).

L'invention concerne également une nanoparticule comprenant au moins un nanocristal organique fluorescent enrobé d'une coquille inorganique, ledit au moins un nanocristal n'émergeant pas à la surface de la coquille.

Selon un mode de réalisation particulier, ladite nanoparticule est susceptible d'être obtenue selon le procédé de l'invention. Ces nouveaux nanocomposés fluorescents peuvent par exemple être utilisés comme traceur ou capteur chimique.

Selon un mode de réalisation particulier de l'invention, ladite nanoparticule comprend un coeur cristallin organique fluorescent enrobé d'une coquille inorganique (c'est-à-dire, nanoparticule "coeur-coquille"). S'agissant d'une nanoparticule, le coeur cristallin est de taille nanométrique (c'est-à-dire, d'une taille nécessairement inférieure à la taille des nanoparticules définie ci-dessous). Autrement dit, il s'agit d'un nanocristal.

On pourra parler de nanoparticules à coeur cristallin « pur » par opposition aux nanoparticules rapportées par Larson et coll. par exemple, dont le coeur est constitué de molécules organiques fluorescentes dispersées dans ou greffées à une matrice silicatée **(ref 4).** Le coeur cristallin « pur » des nanoparticules de l'invention permet une meilleure photostabilité et luminosité.

Selon un mode de réalisation plus particulier, le coeur cristallin de la nanoparticule selon l'invention peut être monocristallin. En effet, de façon surprenante, le procédé de la présente invention permet d'obtenir des nanoparticules comprenant un coeur monocristallin, c'est à dire un coeur constitué d'un unique cristal homogène, dont les plans réticulaires ont une orientation uniforme dans tout le volume du cristal. La monocristalinité du coeur des nanoparticules de l'invention leur confère un comportement d'émetteur unique de fluorescence et permet d'envisager de nouvelles applications des nanoparticules en tant que capteur ultrasensible ou à très fort contraste d'intensité de fluorescence en fonction de l'environnement chimique ou biologique. En effet, dans le cas des monocristaux, le comportement d'émetteur unique est très avantageux pour la réalisation de fonction de signalisation de capteurs (ne présentant que deux états : l'un correspondant à une intensité nulle (0) l'autre où le nanocristal est fluorescent (1 détection)). A l'inverse, des nanoparticules constituées d'un coeur polycrystallin (c'est-à-dire constitué de plusieurs nanocristaux) n'ont pas de caractère émetteur unique mais celui-ci sera fonction de l'environnement. Cela se traduit par la fluorescence d'une partie des nanocristaux tandis que les autres restent éteints selon les conditions, et donc par des évolutions d'intensité de fluorescence au lieu de comportements bien distincts et facilement décelables (état « 0 » ou « 1 »), ce qui conduit à une perte de contraste très importante et de sensibilité en fonction de l'environnement chimique ou biologique.

On entend par « nanoparticule » au sens de la présente invention, une particule de dimension nanométrique. Par exemple, la nanoparticule de la présente invention peut présenter un diamètre inférieur ou égal à 1 µm. Par exemple, la nanoparticule peut présenter un diamètre allant de 20 nm (nanomètres) à 1 µm, par exemple de 20 à 800 nm, par exemple de 20 à 600 nm, par exemple de 20 à 200 nm, par exemple de 20 à 100 nm.

Avantageusement, la nanoparticule de la présente invention peut être parfaitement sphérique. En effet, la géométrie sphérique et les bonnes propriétés de surface des nanoparticules de la présente invention, notamment en termes de très faible rugosité de surface (de l'ordre de 0,1 à 0,5 nanomètres) et d'hydrophylicité, améliorent leurs propriétés optiques, en plus de favoriser leur biocompatibilité et leur cheminement dans le milieu biologique.

On entend par « nanocristal » au sens de la présente invention un cristal de dimension nanométrique comprenant au moins un composé organique fluorescent défini précédemment. Par exemple, le nanocristal selon l'invention peut présenter un diamètre inférieur ou égal à 1 µm. Par exemple, le nanocristal peut présenter un diamètre allant de 10 nm à 1 µm, par exemple de 20 à 700 nm, par exemple de 20 à 500 nm, par exemple de 20 à 400 nm, par exemple de 20 à 300 nm, par exemple de 10 nm à 200 nm, par exemple de 10 nm à 100 nm. En effet, les inventeurs ont pû élaborer des nanocristaux de plusieurs dizaines de nm de diamètre encapsulés dans des sphères inorganiques avec des distributions de taille de particules relativement étroites. Les nanocristaux de taille supérieure à 10 nm présentent l'avantage d'éviter que les particules soient diffusibles au travers des vaisseaux sanguins, notamment de la souris. Le nanocristal est également appelé « cristallite organique » dans le présent texte. Il est entendu que le nanocristal étant enrobé d'une coquille inorganique pour constituer la nanoparticule, la taille du nanocristal est nécessairement inférieure à celle définie pour les nanoparticules ci-dessus.

Le nanocristal se trouvant au coeur de la nanoparticule, on pourra se référer au « coeur cristallin » pour désigner le nanocristal. Les deux termes sont utilisés de manière interchangeable dans la présente demande.

Le coeur cristallin (ou nanocristal) fluorescent de la présente invention peut comprendre une pluralité de molécules de fluorophore. De préférence, le nanocristal selon l'invention peut comprendre un grand nombre de molécules de fluorophore lui conférant un coeur organique fortement fluorescent. Le nombre de molécules comprises dans le nanocristal dépend de la taille des molécules et de la taille du nanocristal. Selon l'invention, le nanocristal peut comprendre jusqu'à 10¹⁰ molécules de fluorophore, de préférence jusqu'à 10⁸, par exemple de 10³ à 10⁸ molécules, par exemple de 10⁴ à 10⁷, par exemple de 10⁴ à 10⁶ molécules de fluorophore.

On entend par « coquille inorganique » selon la présente invention, une coquille d'oxyde métallique. Selon l'invention, la coquille inorganique peut être choisie dans le groupe comprenant une coquille silicate, titanate, zirconate, stanate, borate, aluminate et yttriate. La coquille inorganique permet d'éviter que les cristallites organiques soient en contact direct avec le milieu extérieur. En outre, elle présente l'avantage d'être inerte, biocompatible « in vivo » et de rendre la nanoparticule de la présente invention plus furtive. En effet, la coquille inorganique confère à la nanoparticule un caractère hydrophile crucial, notamment pour les applications en imagerie médicale. De plus, la coquille permet de moduler aisément la balance hydrophile-hydrophobe de la nanoparticule hybride et d'ajuster ainsi ses interactions dans l'organisme. En effet, un caractère très hydrophile facilite la dispersion de ces particules dans des solutions physiologiques et leur injection dans le flux sanguin. Ce caractère hydrophile marqué réduit significativement « in vivo » toutes interactions de ces nanoparticules avec les protéines permettant d'obtenir dans ce cas des traceurs lumineux « furtifs ». A l'inverse, on peut également envisager de réduire le caractère hydrophile de la coquille inorganique en utilisant des alcoxydes métalliques (ou précurseurs sol-gel) qui présentent une fonction organique, par exemple un groupement méthyl (-CH₃) ou éthyl (-C₂H₅) non hydrolysable et que l'on retrouvera ainsi en surface des particules. Ce caractère hydrophile moins marqué autorisera des interactions entre traceurs et certaines protéines qui pourraient être mise en évidence.

Selon un mode de réalisation particulier de l'invention, la coquille inorganique peut être une coquille silicatée. La coquille silicatée présente l'avantage d'être inerte, amorphe, biocompatible et transparente dans le visible et le proche IR. Cette transparence dans le visible et le proche IR est favorable pour des applications en imagerie. De plus, il est possible de réaliser des coquilles silicatées non poreuses.

Selon l'invention, la coquille inorganique peut être poreuse ou non poreuse.

Selon un mode de réalisation particulier de l'invention, la coquille inorganique peut être poreuse. Par exemple, la coquille inorganique peut être microporeuse ou présenter des tailles de pores inférieures à 5 nm, de préférence inférieures à 2 nm. Ceci permet des interactions entre le nanocristal fluorescent et l'environnement (eaux usées, milieux biologiques) et la détection de petites molécules capables de passer au travers des pores de la coquille. Cette détection est basée sur une variation significative des propriétés de luminescence et applicable par exemple dans le domaine des capteurs chimiques.

Selon un autre mode de réalisation de l'invention, la coquille inorganique peut être non poreuse et donc totalement étanche. En effet, une coquille non poreuse présente l'avantage de stabiliser chimiquement le nanocristal fluorescent contenu au coeur de la coquille en évitant toute réaction des fluorophores avec le milieu extérieur, par exemple avec l'oxygène, conduisant à une photostabilité améliorée. En outre, la forme cristalline des fluorophores contenues dans le nanocristal permet également d'améliorer la photostabilité des nanoparticules de la présente invention.

Selon l'invention, la coquille inorganique peut laisser passer la lumière aux longueurs d'onde visible et IR soit de 400 à 3000 nm au minimum qui couvre ainsi largement le domaine de longueur d'onde de la fluorescence des nanocristaux organiques. En effet, la coquille inorganique est en oxyde métallique et est transparente dans le visible et le proche IR quelque soit le métal. Ainsi, cette transparence est parfaitement adaptée pour les applications visées.

Selon l'invention, la coquille inorganique peut avoir une épaisseur allant de quelques nm à 1 µm. La coquille inorganique peut, par exemple, avoir une épaisseur allant de 1 nm à 1 µm, plus particulièrement de 1 à 200 nm, plus favorable pour des applications en imagerie, par exemple de 2 à 100 nm.

Ainsi, les nanoparticules de la présente invention présentent l'avantage d'être très lumineuses et non-diffusibles au travers des parois des vaisseaux sanguins (propriété indispensable pour l'angiographie) car elles comprennent un très grand nombre de molécules de fluorophores. En effet, ceci augmente considérablement la section efficace d'absorption et ainsi l'intensité de fluorescence de ces nanoparticules (ou l'intensité du rayonnement émis par le nanocristal fluorescent). Ainsi, en augmentant significativement la concentration locale en fluorophores dans les vaisseaux sanguins, ce nouveau type de traceur permet d'obtenir des intensités de fluorescence nettement plus élevées que celles obtenues avec les solutions commerciales actuelles où chaque particule correspond à une seule molécule fluorescente. Ce gain en intensité est de plusieurs ordres de grandeur en section efficace d'absorption des nanocristaux par rapport à une molécule fluorescente, par exemple de 2 à 3 ordre de grandeur grâce à cet effet de concentration en fluorophore. Ce gain est donc susceptible de conduire à une amélioration importante du contraste des images et permet d'augmenter nettement la profondeur accessible par l'imagerie médicale, notamment par la technique de microscopie biphotonique de fluorescence à balayage laser. On peut ainsi espérer réaliser des angiographies cérébrales pour des profondeurs de tissus nettement supérieures au centimètre au lieu de 1 mm de profondeur actuelle.

De plus, les nanoparticules de la présente invention présentent l'avantage d'être photostables. D'une part, la coquille sol-gel stabilise mécaniquement et chimiquement les nanocristaux organiques. D'autre part, la cristallinité des fluorophores organiques leur confère des photostabilités nettement plus élevées que celles généralement observées pour des molécules organiques en solution. En outre, les excitations sont plus localisées dans le cas des cristaux organiques que dans le cas des cristaux semi-conducteurs, ce qui permet d'éviter la perte d'efficacité due à l'interaction entre porteurs (comme par exemple l'effet Auger). Ces bonnes photostabilités permettent au niveau de l'imagerie intra-vitale d'observer visualiser une même zone plusieurs fois au cours du temps, d'observer les évolutions thérapeutiques ou d'étudier la migration de particules de différentes tailles dans des cellules ou tumeurs pour la compréhension de l'efficacité de nanomédicaments (médicaments encapsulés dans des polymères biodégradables de type « cyanoacrylate » par exemple).

Par ailleurs, les nanoparticules de la présente invention sont parfaitement sphériques, ce qui leur confère de très bonnes propriétés de surface, notamment en termes de très faible rugosité de surface et d'hydrophilicité, d'où des propriétés de furtivité nettement améliorées. Notamment, contrairement aux nanoparticules de l'art antérieur qui sont généralement des polyèdres irréguliers de forme globale sphérique, le caractère parfaitement sphérique des particules de la présente invention favorise la furtivité *in vivo* des nanoparticules, par exemple pour leur utilisation comme traceur. Ceci permet par exemple une meilleure diffusion des nanoparticules dans les vaisseaux sanguins et d'éviter l'accumulation des nanoparticules dans certains organes (comme par exemple le foie ou le rein).

En outre, les nanoparticules de la présente invention présentent l'avantage d'être biocompatibles, hydrophiles, et dispersibles en solution physiologique. De plus, leur taille peut être modulée facilement, pouvant aller de quelques dizaines à quelques centaines de nanomètres. Par exemple, des particules hydrophiles, de taille inférieure à 100nm environ, présentent l'avantage de ne pas être ou très peu perturbées par des protéines. En effet, les protéines interagissent in vivo et peuvent se coller sur toute particule étrangère et l'entraîner vers un organe spécifique qui n'est généralement pas la tumeur que l'on veut caractériser. En outre, l'étude de l'influence de la taille des traceurs sur leur capacité d'accès et de migration à l'intérieur de tumeurs permettra de mieux comprendre l'efficacité de certains traitements médicaux.

Ainsi, ces nanomatériaux hybrides organo-minéraux allient les avantages des nanocristaux organiques (intensité de luminescence élevée, photostabilité, effets de taille...) à ceux de la matrice amorphe minérale (stabilité, facilité de mise en forme et transparence).

L'invention concerne également l'utilisation de nanoparticules selon l'invention comme traceur en imagerie médicale. En effet, les caractéristiques particulières des nanoparticules de la présente invention, notamment en terme de luminosité augmentée, de photostabilité améliorée, de biocompatibilité, de taille contrôlée, en font des traceurs très lumineux pour l'imagerie médicale. Les applications peuvent concerner l'homme ou l'animal, y compris le petit animal. Par exemple, l'angiographie cérébrale laser est utilisée sur le petit animal (rat ou souris) afin de tester les nouvelles les thérapies en développement pour l'homme. En effet, l'angiographie en profondeur du cortex cérébral de la souris permet de tester les médicaments et nouvelles thérapies en développement ainsi que les méthodes de diagnostique en développement chez l'homme comme par exemple l'angiographie oculaire laser.

Ainsi, selon un mode de réalisation particulier de l'invention, l'imagerie médicale peut être par exemple l'angiographie cérébrale ou l'angiographie oculaire. Notamment, l'utilisation des nanoparticules de la présente invention excitées par transition à deux photons peut conduire à une meilleure angiographie cérébrale, plus profonde, avec une puissance laser plus faible et moins de chromophores injectés car concentrés sous forme de cristallites dotés d'une coquille totalement biocompatible. Ceci permet de réduire ou d'éliminer les traumatismes occasionnés généralement par ce type d'examens.

La synthèse des nanoparticules selon l'invention présente l'avantage d'être simple et générique, effectuée en une seule étape réactionnelle, et permet d'élaborer différents traceurs en utilisant différentes phases organiques fluorescentes à différentes longueurs d'onde ou actives en optique non linéaire quadratique (nanocristaux noncentrosymétriques). Ainsi, cette méthode présente l'avantage de sélectionner des fluorophores adaptés pour un type d'application particulier, par exemple, de sélectionner des fluorophores émettant dans le rouge afin de bénéficier de la bonne transparence des tissus biologiques dans ce domaine de longueurs d'onde.

Selon un mode particulier de réalisation de l'invention, l'invention concerne également l'utilisation de nanoparticules selon l'invention comme capteur chimique. En effet, la coquille inorganique d'une nanoparticule selon l'invention peut permettre, en fonction de sa porosité et de la taille de ses pores, le passage de molécules présentes dans le milieu dans lequel est placé la nanoparticule. Ainsi, la coquille inorganique poreuse d'une nanoparticule selon l'invention peut permettre des interactions entre le nanocristal fluorescent et les molécules du milieu extérieur. Ces interactions peuvent engendrer une variation significative des propriétés de luminescence qui permet la détection des molécules du milieu extérieur. A titre d'exemple de mise en oeuvre pour l'utilisation de nanoparticules comme capteur chimique, on peut citer par exemple le document de Burns et al., Small, vol. 2 (2006), 723-726 **(ref 10),** qui décrit des billes de silice comprenant des molécules organiques fluorescentes fonctionnant comme capteur de pH. Cependant, la synthèse de ces billes ne conduit pas à la cristallisation de la phase fluorescente. Le coeur cristallin de la nanoparticule de la présente invention présente l'avantage de contenir un plus grand nombre de molécules organiques fluorescentes (comme décrit précédemment). Ce nombre élevé de molécules conduit à des sections efficaces d'absorption très élevés et donc à des intensités de fluorescences très élevées. Les interactions du coeur cristallin de la nanoparticule de l'invention avec des molécules du milieu extérieur peuvent donc engendrer des variations importantes de fluorescence, d'où l'obtention de capteurs chimiques très efficaces. Pour l'utilisation des nanoparticules de l'invention comme capteur chimique, le coeur cristallin peut comprendre toute molécule organique fluorescente ou complexe de terre rare présentant des intensités très élevées de fluorescence excitées à deux photons est utilisable (comme par exemple les fluorophores précédemment cités).

D'autres avantages pourront encore apparaître à l'homme du métier à la lecture des exemples ci-dessous, illustrés par les figures annexées, donnés à titre illustratif.

### Brève description des figures

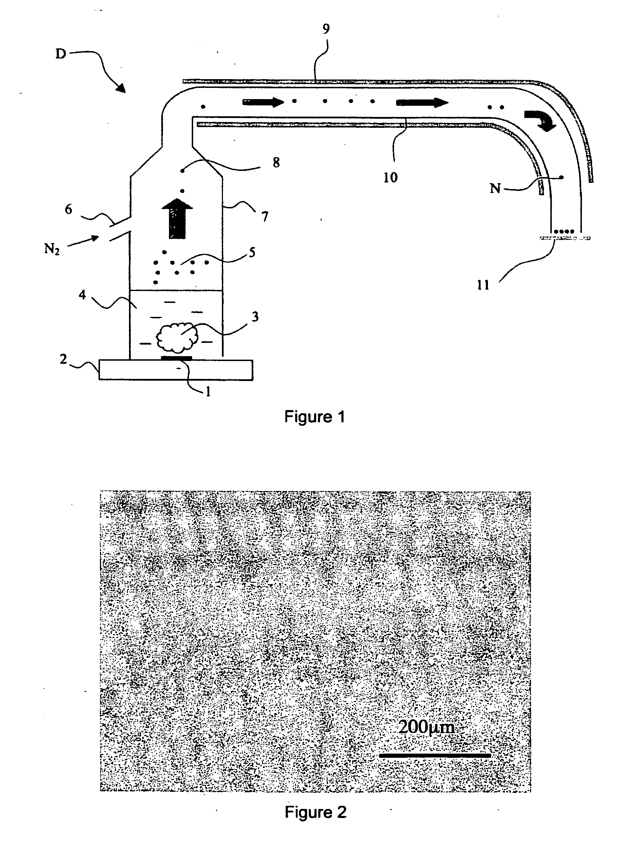

- La figure 1 représente un dispositif D permettant de mettre en oeuvre le procédé de l'invention et d'obtenir les nanoparticules (N) de l'invention. Sur le schéma, (1) représente une céramique piézoélectrique permettant d'exciter la solution, (2) représente le socle du réacteur et (3) représente le phénomène de cavitation provoqué par les ultrasons. La solution (4) est nébulisée en aérosol (5) constitué de gouttelettes (8). L'entrée (6) permet la circulation du gaz qui entraîne les gouttelettes (8) vers une zone de séchage (10) entourée d'une résistance chauffante (9). Les nanoparticules (N) sont ensuite collectées au moyen d'une plaque de verre (11).
- La figure 2 représente la visualisation par microscopie optique des nanoparticules collectées sur lames de verres selon l'exemple 1.
- La figure 3 représente les deux images superposées en microscopie optique confocale d'une même zone de lame de verre comprenant des nanoparticules obtenues selon de l'exemple 1 : l'une des image est obtenue en mode réflexion, et l'autre en mode fluorescence.
- La figure 4 représente des nanoparticules obtenues selon de l'exemple 1 observées au MEB (Microscopie Electronique à Balayage), tailles comprises entre 100 et 600 nm de diamètre.
- La figure 5 représente une nanoparticule obtenue selon de l'exemple 1 observée au MET (Microscopie Electronique en Transmission).
- La figure 6 représente l'analyse chimique EDS (« Energy Dispersive Spectroscopy » ou spectroscopie dispersive en énergie), réalisée sur les nanoparticules hybrides organo-silicatées obtenues selon de l'exemple 1. L'intensité I des raies observées est représentée en fonction de l'énergie E des rayonnements RX enregistrés (en keV).
- La figure 7 représente un cliché de diffraction électronique enregistré à température ambiante sur une nanoparticule.
- La figure 8 représente un coeur monocristallin, issu d'une nanoparticule d'environ 1 micron, débarrassé de sa coquille (obtenu par nébulisation pneumatique puis dissolution de la coquille selon l'exemple 3) observé au MEB (Microscopie Electronique à Balayage). Les coeurs obtenus ont des tailles de l'ordre de 500 à 600 nm de diamètre.
- La figure 9 représente un coeur nanocristallin, issu d'une nanoparticule d'environ 500 nm, débarrassé de sa coquille (obtenu par nébulisation pneumatique puis dissolution de la coquille selon l'exemple 3) observé au MEB (Microscopie Electronique à Balayage). Les coeurs obtenus ont des tailles de l'ordre de 200 à 300 nm de diamètre.

### EXEMPLES

### Exemple 1 : Synthèse de nanocristaux de rubrène enrobés d'une coquille silicatée.

Dans cet exemple, le rubrène est choisi pour l'élaboration de nanocristaux organiques inclus dans des sphères de silice. Le rubrène présente une luminescence orange-rouge (bande de longueur d'onde de 550 à 600 nm) pour laquelle les tissus biologiques présentent une bonne transparence.

La synthèse des nanoparticules de la présente invention selon le procédé de la présente invention est illustrée par la figure 1 qui représente le réacteur (7) comprenant un mélange sol-gel (4). Le mélange sol-gel (4), après nébulisation, conduit à l'aérosol (5) constitué de gouttelettes (8). L'entrée de gaz (6) permet le transfert de l'aérosol vers la zone (10) de séchage de l'aérosol entourée d'une résistance chauffante (9). Une fois séchées, les nanoparticules (N) de l'invention sont collectées à l'aide d'une plaque de verre (11).

### Mode opératoire pour la préparation du mélange sol-gel (4) :

La poudre microcristalline de rubrène (0,18 g ; 3,3 mmoles ; commercialisée par la société Aldrich) a été dissoute dans une solution contenant 138,75 mL de tétrahydrofurane, 4,16 mL (1,5 mmole) de tétraméthoxysilane (TMOS) et 4,03 mL (1,5 mmoles) de méthyltriméthoxysilane (MTMOS).

L'eau et l'HCL ont été introduits simultanément sous la forme d'une solution diluée d'acide chlorhydrique 0,1 M de volume 3,56 mL. L'eau permet d'activer les réactions d'hydrolyse et de polycondensation des alkoxydes de silicium conduisant par la suite, lors de l'évaporation du THF, à la formation de la coquille silicatées. Après l'ajout de cette solution acide, le pH du mélange est autour de 1.

La solution a ensuite été stockée pendant 1 mois afin de bien avancer les réactions d'hydrolyse et de condensation des alcoxydes.

### Nébulisation du mélange sol-gel (4) et séchage:

La solution (4) homogène et stable a ensuite été atomisée par ultrasons afin d'obtenir un aérosol (5) constitués de gouttelettes (8) présentant des distributions de tailles étroites (figure 1). La nébulisation a été effectuée à l'aide d'un réacteur de nébulisation piézoélectrique (fourni par la société artisanale RBI, France) fonctionnant à une fréquence de 2,1 MHz pour nébuliser la solution (4) avec des tailles de gouttelettes d'environ 2 microns (µm).

L'aérosol a ensuite été transportés sous un flux d'azote (2-3 L/min) vers une zone (10) chaude (à la température de 150°C) afin d'évaporation rapidement le solvant organique et d'initier dans chaque gouttelette la polymérisation du réseau silicaté.

Les nanoparticules (N) ont été collectées en sortie de la zone (10) de séchage du réacteur directement sur des lames de verre (11) disposées perpendiculairement au flux d'azote qui transporte l'aérosol (figure 1). Les lames de verres sont rapidement devenues blanchâtres à cause du dépôt des particules silicatées.

0,53 grammes de nanoparticules ont ainsi été obtenues (ce qui correspond à un rendement de l'ordre de 25%). Ce rendement faible est du au mode de collection peu efficace des particules (en sortie de zone de séchage sur des lames de verres).

L'invention est décrite dans ce qui précède à titre d'exemple. Il est entendu que l'homme du métier est à même d'utiliser différents moyens de réalisation de l'invention sans pour autant sortir du cadre de l'invention. En outre, dans un mode de réalisation non illustré, les particules peuvent être collectées au moyen d'un filtre électrostatique (Joffin et al., J. of Luminescence 113 (2005) 249-257 **(ref 9)).** On peut également envisager de tirer sous vide et de récupérer les particules après séchage à l'aide d'une cartouche filtrante. En optimisant ces deux systèmes de collecte de particules, le rendement du procédé peut atteindre des taux proches de 100%.

### Caractérisation :

Après la synthèse, les nanoparticules de la présente invention peuvent être caractérisées selon différentes techniques.

Tout d'abord, une caractérisation macroscopique a été effectuée par microscopie optique classique et microscopie optique de fluorescence afin de préciser l'homogénéité globale des particules de la synthèse. Les images obtenues par microscopie optique ont confirmé la présence de particules submicroniques blanches, d'aspect homogène, sans aucune ségrégation des phases silicatée et organique (figure 2). De plus, sous exposition UV le dépôt blanc de particule est devenu rouge orangé, très homogène en intensité ce qui confirme l'homogénéité des particules hybrides organo-minérales.

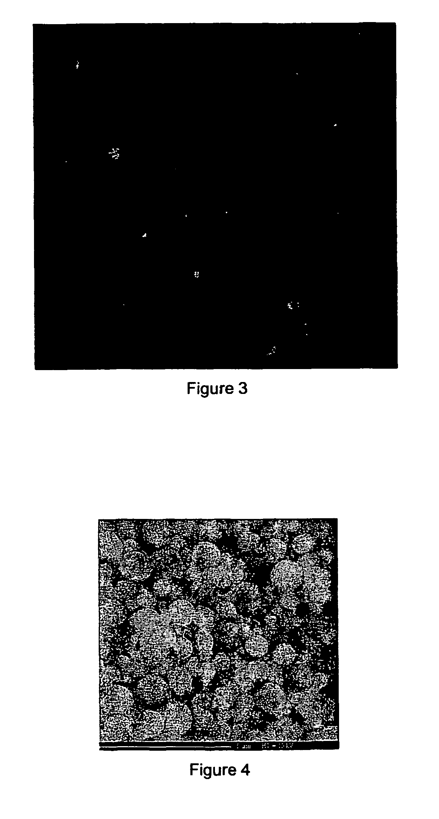

Puis, les particules collées en surface de lame de verre ont ensuite été observées par microscopie optique confocale en modes réflexion et fluorescence. En mode réflexion, on observe des particules de taille de l'ordre de quelques centaines de nm qui semblent sphériques malgré les distorsions (interférences) optiques qui déforment un peu les images et quelques bulles d'air piégées dans le liquide d'indice utilisé pour cette microscopie optique. En mode fluorescence, on observe que toutes ces particules deviennent luminescentes. La figure 3 représente la superposition des deux images de microscopie optique confocale l'une obtenue en mode réflexion et l'autre enregistrée en mode fluorescent. Elle confirme bien que toutes les particules observées sont fluorescentes avec des intensités très élevées compte tenu du nombre élevé de molécule de rubrène contenu dans ces particules isolées.

Les nanoparticules obtenues ont ensuite été analysées en microscopie électronique à balayage (MEB, figure 4) afin de préciser la taille et la distribution de taille des particules. On observe des particules parfaitement sphériques qui présentent une distribution de taille étendue entre 100 et 600 nm mais avec une large majorité de particules autour de 400 à 500 nm due aux premières conditions de nanocristallisation qui sont perfectibles :
- transport de l'aérosol sous flux laminaire afin de réduire la coalescence des gouttelettes,
- taux de solvant plus élevé pour réduire la taille des particules
- optimisation d'une zone de séchage plus rapide de l'aérosol (température, atmosphère contrôlée...)
- amélioration des conditions de récupération des particules (filtre électrostatique, cartouches filtrantes + pompe...).

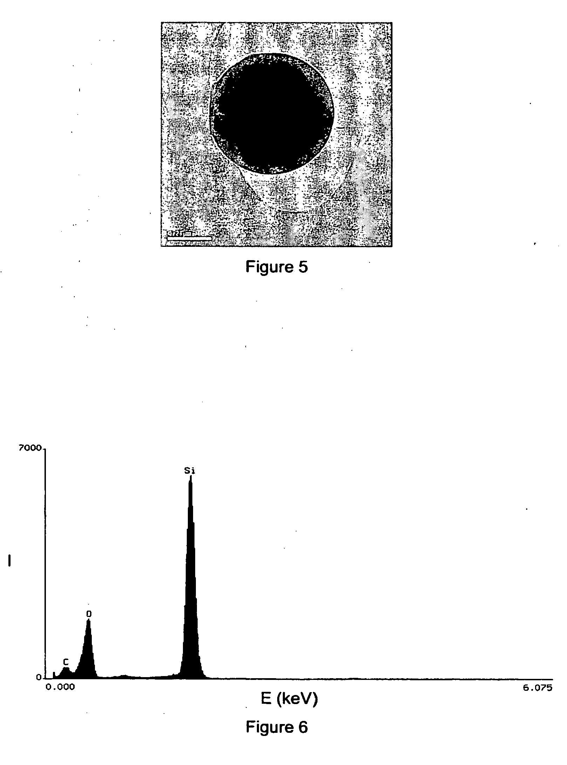

L'analyse chimique EDS (« Energy Dispersive Spectroscopy ») a été réalisée sur les nanoparticules hybrides organo-silicatées obtenues selon de l'exemple 1 et est représentée en figure 6 avec en abscisse l'énergie des rayonnements RX enregistrés (E en keV) et en ordonnée l'intensité de chaque raie (I en coups enregistrés sur le détecteur). On observe les raies K (alpha) caractéristiques des atome de Si et O correspondant bien à la coquille silicatée ainsi que la raie K (alpha) du carbone correspondant au coeur de la particule (nanocristal organique de rubrène). Ainsi l'analyse chimique par EDS au MEB confirme bien la présence d'une coquille silicatée et a permis d'observer la présence des éléments Si, O et C ce qui confirme l'élaboration de nanoparticules hybrides organo-silicatés.

Enfin, les nanoparticules hybrides ont été caractérisées par microscopie électronique en transmission (MET). Les premières observations MET (figure 5) confirment la forme parfaitement sphérique de la nanoparticule, et la taille préalablement observées au MEB. Cette visualisation montre bien la sphéricité des particules obtenues par ce procédé avec une rugosité très faible de quelques angströms (0,1 à 0,5 nm) de la coquille inorganique qui se trouve en surface de particule. La zone plane à gauche de la particule (en noir sur la figure 5), correspond au contact de la nanoparticule en cours de séchage avec la lame de verre qui a servi à récupérer les particules lors des premiers essais. Un séchage plus poussé et un mode de collection plus approprié (filtre électrostatique ou cartouche filtrante) peuvent permettre d'éviter de type de défaut et d'obtenir ainsi des particules totalement sphériques.

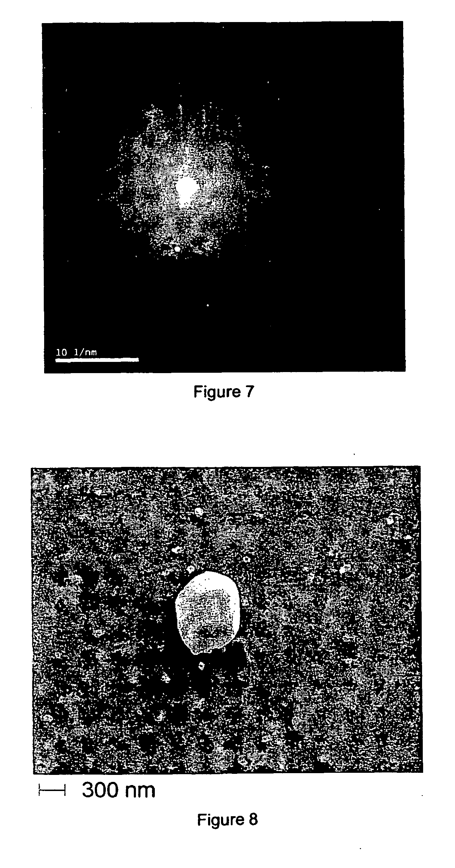

Puis, la cristallinité et la structure des nanocristaux situés au coeur des particules hybrides ont été caractérisées par microscopie en transmission en mode diffraction. Les inventeurs ont récemment maîtrisé une technique de diffraction électronique MET de type « faible dose » (ou « Low dose ») qui permet de travailler sous faible courant d'électron et à basse température (N₂ liquide) afin de réduire sensiblement la dégradation des nanocristaux organiques sous le faisceau d'électron. En effet, les premiers enregistrements ont été perturbés par la dégradation des nanocristaux sous le faisceau d'électrons focalisés en mode diffraction. Ce comportement évanescent est typique de la dégradation de nanocristaux organiques comme les inventeurs l'ont déjà observé lors de l'étude de nanocristaux en couches minces comme décrit dans le document A. Ibanez et al. J. Nanoparticle Research 10 (2008), 129-139 **(ref 11)**. Les premiers tests de diffraction ont tout de même permis d'enregistrer, sous faible courant d'électron, plusieurs images à température ambiante et de prouver la bonne cristallinité des nanocristaux organiques : la figure 7 représente un cliché de diffraction électronique enregistré à température ambiante sur une nanoparticule. Ceci prouve bien la présence du coeur organique cristallin (nanocristal). En effet, par le procédé sol-gel utilisé, on ne forme que des silicates amorphes qui ne produisent aucun phénomène de diffraction. Ces premiers clichés de diffraction sont remarquables car c'est la première fois que des taches de diffraction électronique ont pu être observées à température ambiante pour des nanocristaux organiques. Ces premiers diffractogrammes sont donc très encourageants car ils démontrent la bonne monocristallinité des nanocristaux organiques (un seul nanocristal par nanoparticule). En effet, on n'observe pour chaque particule qu'un seul diagramme de diffraction, constitué de taches très ponctuelles. De plus, la taille de ces nanocristaux est supérieure à 40nm de diamètre ce qui est très encourageant pour l'élaboration de traceurs très lumineux constitués d'un nombre élevé de chromophores. L'abaissement de température permet d'enregistrer de plus nombreuses taches de diffraction, d'exploiter correctement leurs intensités relatives et de caractériser plus finement la structure et la cristallinité de ces taches.

Cette taille moyenne des nanocristaux organiques, situés au coeur des nanoparticules, peut être déterminée plus précisément en testant plusieurs types de techniques de caractérisation comme la diffraction des RX à haute résolution (largeur de raies) pour les particules de petite tailles ainsi que par diffusion des RX ou diffusion inélastique de lumière. La diffraction des RX à haute résolution est une mesure essentielle et complémentaire par rapport aux microscopies électroniques (MEB et MET) qui permettent de visualiser uniquement la totalité des particules hybrides (coeur organique et coquille silicatée). Ceci nécessite de bien maîtriser les conditions de nanocristallisation conduisant à un bon rapport diamètre des nanocristaux / diamètre de la particule hybride. Les nanoparticules de la présente invention peuvent également être caractérisées par diffusions des RX aux petits angles et par diffusion inélastique de la lumière.

### Exemple 2: Synthèse de nanocristaux de cyano-méthoxy-nitro-stilbène (CMONS) enrobés d'une coquille d'oxyde de titane.

Le mode opératoire de l'exemple 1 est reproduit avec, dans cet exemple, un mélange sol-gel (4) préparé à partir de :
- 0,65 g (soit 2,32 mmole) de cyano-méthoxy-nitro-stilbène (CMONS) ;
- 140 mL (soit 1,7 mole) de solvant tétrahydrofurane (THF) ;
- 10,06 mL (soit 3,3.10⁻² mole) d'isopropoxyde de titane comme précurseur sol-gel ;
- 2,46 mL d'une solution d'acide chlorhydrique dans l'eau (de concentration 0,1 M), soit 0,14 mole d'eau.

Après nébulisation du mélange sol-gel et séchage de l'aérosol selon la méthode décrite dans l'exemple 1, les nanoparticules de titanate sont collectées avec un rendement de l'ordre de 30% en masse de nanoparticules.

### Exemple 3 : Synthèse de nanocristaux de cyano-méthoxy-nitro-stilbène (CMONS) enrobés d'une coquille silicatée.

Dans cet exemple, les conditions de synthèse des nanoparticules selon l'invention sont les suivantes :
- la matrice silicatée est constituée à 50% de tétraméthoxysilane (TMOS, Si(OCH₃)₄) et à 50% de méthyltriméthoxysilane (MTMOS, CH₃Si(OCH₃)₃),
- la quantité de chromophore (CMONS) utilisée pour la synthèse (ou rapport de la concentration de CMONS sur la concentration d'alkoxyde de silicium) est d = [CMONS] / [alkoxyde de silicium] = 0,05.
- le taux de dilution (rapport de la concentration de solvant sur la concentration d'alkoxyde de silicium) est s = [solvant] / [alkoxyde de silicium] = 50 ;
- la catalyse acide est effectuée à un pH inférieur à 2 ;
- le taux d'hydrolyse (rapport de la concentration d'eau sur la concentration de fonctions alcoxydes (―OR²) provenant des alcoxydes métalliques TMOS et MTMOS) est h = [H₂O] / [―OR²] = 1 (les concentrations sont calculées sur la base du mélange initial, autrement dit, le procédé est ici mis en oeuvre avec autant de molécules d'eau que de fonctions alcoxydes (―OR²) du composé de formule (I)) ;
- le solvant utilisé est le THF ;
- le volume totale de la solution est fixé à 240mL

### a) Préparation du mélange sol-gel :

Le mode opératoire de l'exemple 1 pour la préparation du mélange sol-gel a été réalisé avec les proportions suivantes :
- 780,4 mg de α-[(4'-méthoxyphényl)méthylène]-4-nitro-benzèneacétonitrile (ou CMONS) ;
- 228,4 mL de solvant THF (tétrahydrofurane) ;
- 4,112 mL de tétraméthoxysilane (TMOS) et 3,976 mL de méthyltriméthoxysilane (MTMOS) comme précurseurs sol-gel ;
- 3,512 mL d'une solution d'acide chlorhydrique dans l'eau (de concentration 0,1 M).

Cette solution a ensuite été placée dans une étuve à 50°C pendant 4,5 jours afin de lancer l'hydrolyse et la condensation des alcoxydes (TMOS et MTMOS). Puis, elle a été stockée à température ambiante (de 15 à 25°C) durant 20 jours à l'abris de la lumière afin de faire vieillir la solution (c'est-à-dire de bien avancer les réactions d'hydrolyse et de condensation des alcoxydes).

Après vieillissement, le mélange a été vaporisé par nébulisation pneumatique.

### b) Synthèse des nanoparticules par nébulisation pneumatique :

Le mélange sol-gel ci-dessus a été atomisé par nébulisation pneumatique où le mélange est fragmenté sous pression de gaz arrivant perpendiculairement par un gicleur, permettant l'obtention d'un aérosol constitué de gouttelettes de taille d'environ 1 à 2 micron. La nébulisation a été effectuée en 4 heures à l'aide d'un réacteur de nébulisation pneumatique (atomiseur modèle 3076 commercialisé par la société TSI INC., U.S.A.) avec les paramètres de nébulisation suivants :
- Température du four : T_{four} = 150°C ;
- Température du filtre électrostatique : T_{filtre électrostatique} = 140°C
- Pression du gaz (diazote) : P_{N2} = 1,8 bars ;
- Débit de l'air comprimé (en litre par heure) : D_{air comprimé} = 2,5 Uh.

L'aérosol a ensuite été transporté sous flux gazeux et séché selon la méthode décrite dans l'exemple 1. La collection des nanoparticules est réalisée en sortie de la zone de séchage grâce à un filtre électrostatique réalisé par application d'une tension de 10 kV (kilovolt). Ainsi, 2,2 grammes de nanoparticules ont été obtenues (ce qui correspond à un rendement de l'ordre de 50%). En optimisant les conditions et la collecte des particules, le rendement du procédé peut atteindre des taux proches de 100%.

### c) Séparation des particules par centrifugation :

La poudre de nanoparticules obtenue après nébulisation est polydisperse. Les grosses particules ont donc ensuite été séparées des petites par centrifugation.

Pour cela, 15mg de poudre ont été placés dans 30 mL d'eau déminéralisée. La solution a été sonifiée afin de bien disperser la poudre dans l'eau. Puis, on procède à la centrifugation.

La centrifugation a ensuite été réalisée pendant 30 secondes à 7800 tours/min pour séparer les plus grosses particules (800 - 1000 nm). Celles-ci se retrouvent ainsi « couchées » au fond du tube récolté.

Le surnageant a été récupéré afin de le recentrifuger 5 min à 5000 tours/min pour obtenir cette fois les particules de tailles moyennes (400 - 700 nm). Ces particules se retrouvent ainsi au fond du tube récolté.

Les différents flacons de particules obtenus ont ensuite été traités séparément afin de mettre à nu le coeur des nanoparticules de l'invention, permettant de démontrer sa monocristallinité.

### d) Mise en évidence du coeur unique monocristallin :

### - cas de nanoparticules obtenues par nébulisation pneumatique, présentant un diamètre de 800 nm à 1 micron.

Ces nanoparticules (15 mg) ont ensuite été introduites dans 20 mL une solution de NaOH à 10⁻²M et le tout a été mis sous agitation magnétique pendant une semaine à température ambiante (15 à 25°C), conduisant à des particules cristallines pures de CMONS.

De cette manière, il a été démontré que les nanoparticules comprenant un coeur de CMONS enrobés d'une coquille silicatée selon l'invention, sont constituées d'un seul nanocristal pur de CMONS et que celui-ci est monocristallin. L'analyse au MEB des nanocristaux de CMONS montre que ces monocristaux ont un diamètre d'environ 500 à 600 nm (Figure 8).

### - cas de nanoparticules obtenues par nébulisation pneumatique, présentant un diamètre de 400 nm à 700 nm.

Ces nanoparticules (20 mg) ont ensuite été introduites dans 40 mL d'une solution de NaOH à 10⁻³M et le tout a été mis sous agitation magnétique pendant trois semaines à température ambiante (15 à 25°C), conduisant à des particules cristallines pures de CMONS.

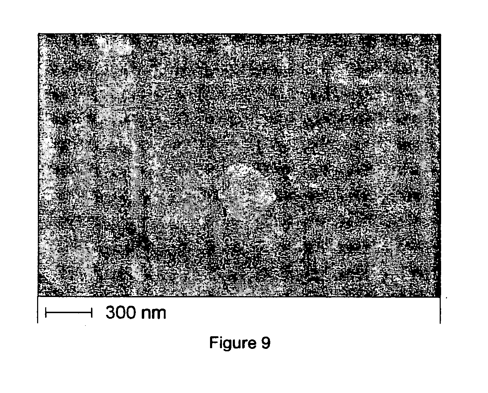

De cette manière, il a été démontré que les nanoparticules comprenant un coeur de CMONS enrobés d'une coquille silicatée selon l'invention, sont constituées d'un seul nanocristal pur de CMONS et que celui-ci est monocristallin. L'analyse au MEB des nanocristaux de CMONS montre que ces monocristaux ont un diamètre d'environ 200 à 300 nm (Figure 9).

### Listes des références

(1) « Water-soluble quantum dots for multiphonon fluorescence imaging in vivo » D.R. Larson et al. Sciences 300 (2003), 1434-1436.
(2) « Blue luminescence from (CdS)ZnS core-shell nanocrystals » J.S. Steckel et al. Ang. Chem. Inter. Ed. 43 (2004), 2154-2158.
(3) « In vivo imaging of quantum dots encapsuled in phospholipids micelles » B. Dubertret et al. Sciences 298 (2002), 1759-1762.
(4) « Bright and stable Core-shell fluorescent silica nanoparticles » H. Ow, D. R. Larson, M. Srivastava, B. A. Baird, W. W. Webb, U. Wiesner. Nano Letters, vol 5, n°1, 113-117 (2005).
(5) C.J. Brinker et G.W. Scherer, Sol-Gel Science, The Physics and Chemistry of SolGel Processing, Academic Press, New York, 1990.
(6) Avnir D., Kaufman V.R., Reisfeld R., J. Non. Cryst. Solids, 1985, 74, 395-406.
(7) C. Sanchez and F. Ribot, New J. Chem., 1994, 18, 1007-1047.
(8) Brevet FR n° 2 853 307
(9) N. Joffin, J. Dexpert-Ghys, M. Verelst, G. Baret, A. Garcia, J. of Luminescence 113 (2005) 249-257
(10) A. Burns, P. Sengupta, T. Zedayko, B. Baird and U. Wiesner, Small, Vol. 2 (2006), 723-726.
(11) « TEM characterization of organic nanocrystals grown in sol-gel thin films ». A. Ibanez et al. J. Nanoparticle Research 10 (2008), 129-139.

## Revendications

1. Procédé de préparation de nanoparticules comprenant un coeur cristallin organique fluorescent enrobé d'une coquille inorganique, ledit procédé comprenant les étapes suivantes :
(i) nébuliser un mélange sol-gel susceptible d'être obtenu par un procédé comprenant l'ajout dans au moins un solvant organique, en présence d'eau, d'un composé organique fluorescent et d'au moins un alcoxyde métallique de formule (I) suivante :
R¹ₓM(OR²)_{y} Formule (I)
dans laquelle :
- M est un métal choisi dans le groupe comprenant Si, Ti, Zr, Sn, B, Al, et Y ;
- x est un nombre entier allant de 0 à 2 ;
- y est un nombre entier allant de 1 à 6 ;
- R¹ et R² sont indépendamment un radical organique compatible avec la chimie sol-gel ;
(ii) sécher le mélange sol-gel nébulisé en évaporant le solvant et l'eau présents dans le mélange sol-gel.

2. Procédé selon la revendication 1 réalisé avec au moins une des conditions suivantes :
(a) le composé organique fluorescent est choisi dans le groupe comprenant la famille des polyaromatiques, la familles des stilbènes, la familles des naphtilimides, la famille des rhodamines, la famille des diarylméthanes, la famille des pérylènes diimides, les dérivés du dipyrrométhène difluorure de bore, les complexes de terre rares ;
(b) l'alcoxyde métallique est choisi dans le groupe comprenant le tétraméthoxysilane (TMOS, Si(OCH₃)₄), le tétraéthoxysilane (TEOS, Si(OC₂H₅)₄), le méthyltriméthoxysilane (MTMOS, CH₃Si(OCH₃)₃), l'éthyltriéthoxysilane (ETEOS, C₂H₅Si(OC₂H₅)₃), le 1,2-bis(triméthoxysilyl)ethane (TMSE), le 3-glycidoxypropyl)trimethoxysilane (GPTMS), ou un mélange de ceux-ci ;
(c) le solvant est choisi dans le groupe comprenant les alcools R^{S}-OH où R^{S} est un radical alkyle en C₁ à C₆, les cétones R^{S1}-C(=O)-R^{S2}, les éthers R^{S1}-O-R^{S2}, où R^{S1} et R^{S2} sont indépendamment des radicaux alkyles en C₁ à C₆, le tétrahydrofurane, l'acétonitrile, le diméthylformamide, le toluène, le diméthylsulfoxyde, le dioxane, l'acétone, l'acide acétique, l'acide formique, le dichlorométhane, le chloroforme, le dichloroéthane, l'acétate d'éthyle, le diéthyléther ou un mélange de ceux-ci ;

3. Procédé selon la revendication 1 ou 2 dans lequel le composé organique fluorescent est choisi dans le groupe comprenant le rubrène, le tétracène, le cyano-méthoxy-nitro-stilbène (CMONS), le diéthylamino-nitro-stilbène, le rhodamine B, l'Auramine O et les complexes d'Europium, d'Ytterbium, d'Erbium, de Néodyme.

4. Procédé selon l'une quelconque des revendications 1 à 3 dans lequel l'étape (ii) de séchage est réalisée avec au moins une des conditions suivantes :
- à des conditions de température et de pression permettant l'évaporation du solvant et de l'eau présents dans le mélange sol-gel ;
- sous un flux de gaz sec ;
- en présence d'un matériau permettant de capter le au moins un solvant ;

5. Nanoparticule susceptible d'être obtenue par le procédé selon l'une quelconque des revendications 1 à 4 comprenant un coeur cristallin organique fluorescent enrobé d'une coquille inorganique.

6. Nanoparticule selon la revendication 5 dans laquelle le coeur cristallin est monocristallin.

7. Nanoparticule selon la revendication 5 ou 6 dans laquelle la coquille inorganique a une épaisseur allant de 1 nm à 200 nm.

8. Utilisation de nanoparticule selon l'une quelconque des revendications 5 à 7 comme traceur en imagerie médicale.

9. Utilisation selon la revendication 8 dans laquelle l'imagerie médicale est l'angiographie cérébrale ou l'angiographie oculaire.

10. Utilisation de nanoparticule selon l'une quelconque des revendications 5 à 7 comme capteur chimique.

## Claims

1. A process for preparing nanoparticles comprising a fluorescent organic crystal core coated with an inorganic shell, said process comprising the following steps:
(i) nebulizing a sol-gel mixture that may be obtained via a process comprising the addition of at least one organic solvent, in the presence of water, of one fluorescent organic compound and of at least one metal alkoxide of formula (I) below:
R¹ₓM(OR²)_{y} Formula (I)
in which:
- M is a metal chosen from the group comprising Si, Ti, Zr, Sn, B, Al and Y;
- x is an integer ranging from 0 to 2;
- y is an integer ranging from 1 to 6;
- R¹ and R² independently represent an organic radical that is compatible with sol-gel chemistry;
(ii) drying the nebulized sol-gel mixture by evaporating off the solvent and the water present in the sol-gel mixture.

2. The process as claimed in claim 1, performed with at least one of the following conditions:
(a) the fluorescent organic compound is chosen from the group comprising the polyaromatic family, the stilbene family, the naphthalimide family, the rhodamine family, the diarylmethane family, the perylene diimide family, boron dipyrromethene difluoride derivatives, and rare-earth metal complexes;
(b) the metal alkoxide is chosen from the group comprising tetramethoxysilane (TMOS, Si(OCH₃)₄), tetraethoxysilane (TEOS, Si(OC₂H₅)₄), methyltrimethoxysilane (MTMOS, CH₃Si(OCH₃)₃), ethyltriethoxysilane (ETEOS, C₂H₅Si(OC₂H₅)₃), 1,2-bis(trimethoxysilyl)ethane (TMSE), 3-glycidoxypropyl)trimethoxysilane (GPTMS), or a mixture thereof;
(c) the solvent is chosen from the group comprising the alcohols R^{S}-OH in which R^{S} is a C₁ to C₆ alkyl radical, the ketones R^{S1}-C(=O)-R^{S2}, the ethers R^{S1}-O-R^{S2}, in which R^{S1} and R^{S2} are independently C₁ to C₆ alkyl radicals, tetrahydrofuran, acetonitrile, dimethylformamide, toluene, dimethyl sufoxide, dioxane, acetone, acetic acid, formic acid, dichloromethane, chloroform, dichloroethane, ethyl acetate, diethyl ether or a mixture thereof.

3. The process as claimed in claim 1 or 2, in which the fluorescent organic compound is chosen from the group comprising rubrene, tetracene, cyano-methoxy-nitro-stilbene (CMONS), diethylamino-nitro-stilbene, rhodamine B, auramine O and europium, ytterbium, erbium and neodymium complexes.

4. The process as claimed in any one of claims 1 to 3, in which the drying step (ii) is performed with at least one of the following conditions:
- with temperature and pressure conditions allowing the evaporation of the solvent and the water present in the sol-gel mixture;
- under a stream of dry gas;
- in the presence of a material that can uptake the at least one solvent.

5. A nanoparticle that may be obtained via the process as claimed in any one of claims 1 to 4, comprising a fluorescent organic crystalline core coated with an inorganic shell.

6. A nanoparticle as claimed in claim 5, in which the crystalline core is monocrystalline.

7. The nanoparticle as claimed in claim 5 or 6, in which the inorganic shell has a thickness ranging from 1 nm to 200 nm.

8. The use of the nanoparticle as claimed in any one of claims 5 to 7 as a tracer in medical imaging.

9. The use as claimed in claim 8, in which the medical imaging is cerebral angiography or ocular angiography.

10. The use of the nanoparticle as claimed in any one of claims 5 to 7 as a chemical sensor.

## Patentansprüche

1. Verfahren zur Herstellung von Nanopartikeln, die einen fluoreszierenden organischen kristallinen Kern umfassen, der von einer anorganischen Schale umgeben ist, wobei das Verfahren folgende Schritte umfasst:
(i) Versprühen einer Sol-Gel-Mischung, die durch ein Verfahren erhalten werden kann, das das Hinzufügen von mindestens einem organischen Lösemittel im Beisein von Wasser, einer organischen, fluoreszierenden Verbindung und von mindestens einem Metallalkoxid der nachstehenden Formel (I) umfasst:
R¹ₓM(OR²)_{y} Formel (I)
in welcher :
- M ein aus der Gruppe umfassend Si, Ti, Zr, Sn, B, Al und Y ausgewähltes Metall ist;
- x eine ganze Zahl von 0 bis 2 ist;
- y eine ganze Zahl von 1 bis 6 ist;
- R¹ und R² unabhängig ein organisches mit der Sol-Gel-Chimie kompatibles Radikal sind;
(ii) Trocknen der versprühten Sol-Gel-Mischung durch Verdampfen des in der Sol-Gel-Mischung vorhandenen Lösemittels und Wassers.

2. Verfahren nach Anspruch 1, das mindestens unter einer der folgenden Bedingungen durchgeführt wird:
(a) die fluoreszierende organische Verbindung wird aus der Gruppe ausgewählt, die die Familie der Polyaromaten, die Familie der Stilbene, die Familie der Naphthilimide, die Familie der Rhodamine, die Familie der Diarylmethane, die Familie der Perylendiimide, die Derivate von Dipyrromethenbordifluorid und Metallkomplexe seltener Erden umfasst;
(b) das Metallalkoxid wird aus der Gruppe ausgewählt, die Tetramethoxysilan (TMOS, Si(OCH₃)₄), Tetraethoxysilan (TEOS, Si(OC₂H₅)₄), Methyltrimethoxysilan (MTMOS, CH₃Si(OCH₃)₃), Ethyltriethoxysilan (ETEOS, C₂H₅Si(OC₂H₅)₃), 1,2-bis(Trimethoxysilyl)ethan (TMSE), 3-glycidoxypropyl)trimethoxysilan (GPTMS), oder eine Mischung davon umfasst;
(c) das Lösemittel wird aus der Gruppe ausgewählt umfassend R^{S}-OH-Alkohole, wobei R^{S} ein C₁ bis C₆-Alkylradikal ist, die Ketone R^{S1}-C(=O)-R^{S2}, die Ether R^{S1}-O-R^{S2}, wobei R^{S1} und R^{S2} unabhängig C₁ bis C₆-Alkylradikale sind, Tetrahydrofuran, Acetonitril, Dimethylformamid, Toluen, Dimethylsulfoxid, Dioxan, Aceton, Essigsäure, Ameisensäure, Dichloromethan, Chloroform, Dichloroethan, Ethylacetat, Diethylether oder eine Mischung davon.

3. Verfahren nach Anspruch 1 oder 2, wobei die fluoreszierende organische Verbindung aus der Gruppe ausgewählt wird, die Rubren, Tetracen, Cyanomethoxynitrostilben (CMONS), Diethylaminonitrostilben, Rhodamin B, Auramin O und Europium, Ytterbium, Erbium und Neodymkomplexe umfasst.

4. Verfahren nach einem beliebigen der Ansprüche 1 bis 3, wobei der Trocknungsschritt (ii) unter mindestens einer der folgenden Bedingungen durchgeführt wird:
- unter Temperatur- und Druckbedingungen, die ein Verdampfen des in der Sol-Gel-Mischung vorhandenen Lösemittels und Wassers ermöglichen;
- unter einem Strom von Trockengas;
- bei Vorhandensein eines Werkstoffes, der mindestens das eine Lösemittel aufnehmen kann.

5. Nanopartikel, das durch das Verfahren nach einem beliebigen der Ansprüche 1 bis 4 erhalten werden kann, das einen kristallinen organischen fluoreszierenden Kern umfasst, der von einer anorganischen Schale umgeben ist.

6. Nanopartikel nach Anspruch 5, in welchem der kristalline Kern monokristallin ist.

7. Nanopartikel nach Anspruch 5 oder 6, in welchem die anorganische Schale eine Stärke 1 nm bis 200 nm aufweist.

8. Verwendung eines Nanopartikels nach einem beliebigen der Ansprüche 5 bis 7 als Tracer in der medizinischen bildlichen Darstellung.

9. Verwendung nach Anspruch 8, wobei die medizinische bildliche Darstellung die zerebrale Angiographie oder die Augenangiographie ist.

10. Verwendung eines Nanopartikels als chemischen Sensor nach einem beliebigen der Ansprüche 5 bis 7.
